# EUROPEAN PATENT SPECIFICATION

(11) **EP 0 400 076 B1**
(45) Date of publication and mention of the grant of the patent: **15.05.1996**
(21) Application number: 89903389.8
(22) Date of filing: 17.01.1989
(51) Int. Cl.: C07K 7/08, G01N 33/53

(54) **A SYNTHETIC ANTIGEN EVOKING ANTI-HIV RESPONSE**
SYNTHETISCHES ANTIGEN ZUM BEWIRKEN EINER ANTI-HIV-REAKTION
ANTIGENE SYNTHETIQUE SUSCITANT UNE REACTION ANTI-VIH

(30) Priority: 26.01.1988 US 148692
(43) Date of publication of application: 05.12.1990
(73) Proprietor: THE UNITED STATES OF AMERICA as represented by the Secretary United States Department of Commerce, Springfield, Virginia 22161 (US)
(72) Inventor: BERZOFSKY, Jay, A., Bethesda, MD 20814 (US); TAKAHASHI, Hidemi, Rockville, MD 20852 (US); HOSMALIN, Anne, Bethesda, MD 20814 (US); GERMAIN, Ronald, A., Potomac, MD 20854 (US); MOSS, Bernard, Bethesda, MD 20814 (US)
(74) Representative: Smulders, Theodorus A.H.J., Ir.
(86) International application number: US8900172
(87) International publication number: WO8907112

(56) References cited:
- EP-A- 0 273 716
- WO-A-86/02383
- WO-A-87/02775
- WO-A-87/07616
- Cease, Proceedings of The National Academy of Science, U.S.A., 84 4249-4253, published June 1987
- Delisi, Proceedings of The National Academy of Science, U.S.A., 82 7048-7052, published October 1985
- Hopp, Journal of Immunological Methods, 88: 1-19, published 1986
- Chou, Annual Review of Biochemistry, 47: 251-276, published 1978
- Chou, Biochemistry, 13 (2) 211-221, published 1974
- Rather, Nature, 313: 277-287, published January 1985
- Hopp, Proceedings of The National Academy of Science, U.S.A., 78(6), 3842-3828, published June 1981.

## Description

### BACKGROUND OF THE INVENTION

The present invention is related generally to the field of vaccines and diagnostic or prognostic reagents for AIDS. More particularly, the present invention is related to a synthetic oligopeptide which elicits cytotoxicity by T cells against human immunodeficiency virus (HIV) antigens and proliferation of HIV-specific CTL's.

### STATE OF THE ART

Various strategies are being developed to combat acquired immunedoficiency syndrome (AIDS). Envelope protein of the human immunodeficiency virus (HIV) has been employed as an antigenic agent to generate anti-HIV antibodies. This approach stresses the mechanism of eliciting neutralizing antibodies against the virus.

A different mode of HIV infection, however is the direct cell-to-cell transmission of HIV. Such cell-mediated mechanism does not require de novo infection by extracellular virus. Hence, treatment modalities which depend upon the accessibility and neutralization of the virus by the antibodies, would not be effective in the cases of cell-mediated infection.

It has been reported that cytotoxic T lymphocytes (CTL) play an important role in resistance to viral infection (Kast, et al., J. Exp. Med., 164:723-748 (1986)). However, a synthetic peptide which stimulates increased production of T cells to selectively kill the target (HIV-antigen expressing cells) has not heretofore been known or identified.

EP-A-0273716, filed on 23rd December 1987 and published on 6th July 1988, discloses a method of inducing cellular immunity to native protein of AIDS virus by immunization with short synthetic peptides. Table 3 shows the peptide IleArgIleGlnArgGlyProGlyProArgAlaPheValThrIleGlyLysIleGlyAsnMetArgGlnAlaHisCys. In determining the potential immunogenicity of the protein sequence, the helical amphipathicity of segments along the entire sequence of a protein; the conformational propensity of segments; the presence or absence of helix breakers in segments; and presence and location in the protein sequence of amino acid residues which favor T-cell recognition were considered.

The document teaches T-cell epitopes recognized by helper T-cells in association with class II Major Histocompatibility complex (MHC) molecules. The teachings do not apply to or imply T-cell epitopes recognized by cytotoxic T-cells in association with class I MHC molecules. The citation shows that helper T-cells tend to recognize peptide sequences that can fold as amphipathic helices based on their periodicity of hydrophobicity. This approach is used to locate helper T-cell epitopes on HIV. None of these studies indicate that these principles can be applied to cytotoxic T-cells.

WO 86/02383 discloses antigens of lymphadenopathy associated virus (LAV) and of acquired immunodepressive syndrome (AIDS), a process for producing the antigens, a vaccine composition comprising the antigen, a process for detecting antibodies against LAV or proteins related therewith and diagnostic kits for in vitro detection of antibodies against LAV.

Similarly, WO 87/02775 is directed to a composition and method for detection of and vaccination against viral causative agents of AIDS and AIDS related complex (ARC). Although this document discloses HTLV-III envelope glycoproteins and peptides, it is directed to mechanisms of eliciting antibodies capable of neutralizing HTLV-III.

### SUMMARY OF INVENTION

It is, therefore, an object of the present invention to provide an isolated, synthetic peptide capable of stimulating cytotoxic T cells specifically against HIV antigen-expressing cells. For simplicity, the peptide of the present invention is designated herein as "Env-K₁".

It is a further object of the present invention to provide a pharmaceuticai composition comprising an antigenic amount of the Env-K₁ peptide to stimulate production of HIV-specific CTL.

It is another object of the present invention to provide a diagnostic or-prognostic kit for detecting HIV or predicting the course of HIV infection.

A still further object of the present invention is to provide a use of the Env-K₁ peptide for preparing a pharmaceutical composition for preventing or heating HIV infection in a susceptible host, said composition containing an effective amount of Env-K₁ peptide to, produce HIV-specific CTL.

Other objects and advantages of the present invention will become evident from the detailed description of the invention.

### BRIEF DESCRIPTION OF DRAWINGS

These and other objects, features and many of the attendant advantages of the invention will be better understood upon a reading of the following detailed description when considered in connection with the accompanying drawings wherein:

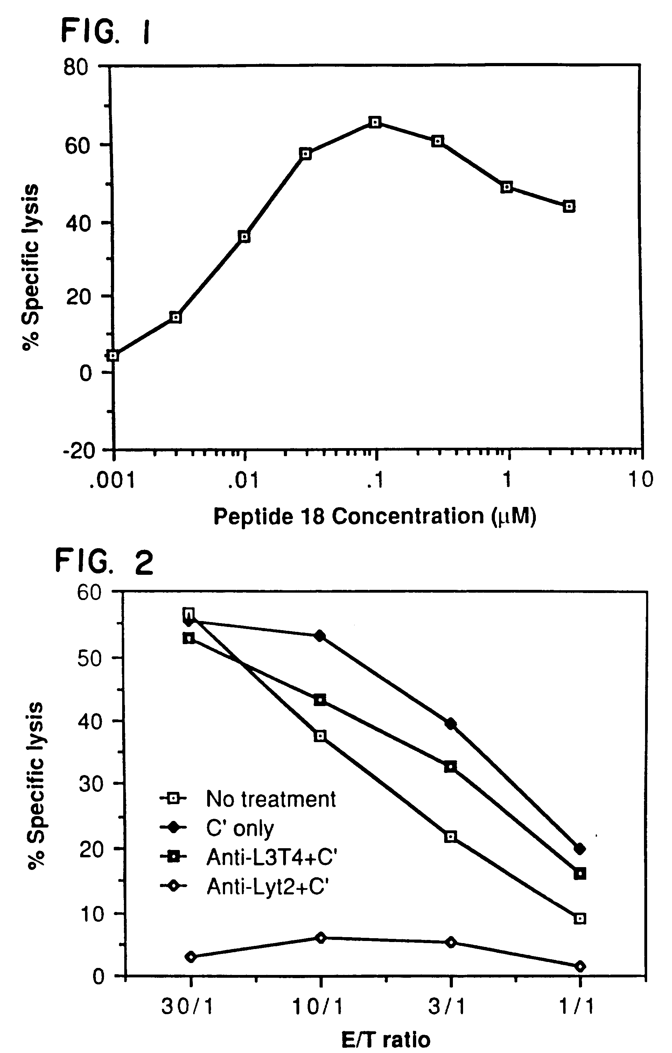

Fig. 1 shows the dose-response of Env-K₁ for sensitization of targets for envelope-specific CTL. Effector CTL line cells (5 x 10⁴/well) were added to the assay culture with various concentration of Env-K₁ in the presence of ⁵¹CR-labelled neo-gene transfected BALB/c3T3 fibroblast (H-2^{d}) target cells (5 x 10³/well). After 6 hr incubation at 37°C, culture supernatant was harvested and radioactivity measured in a gamma counter. Specific ⁵¹Cr-release was calculated as described vide infra. Spontaneous release for the target cells was below 25%.

Fig. 2 shows the phenotype of the H-2^{d} CTL line specific for Env-K₁-bearing cells. 5 x 10³ of ⁵¹Cr-labelled BALB/c3T3 neo-gene transfectants were cultured with cells from the long-term anti-gp160 CTL line at several effector target ratios in the presence of 1 M of Env-K₁. The effector cells were pre-treated with monoclonal antibodies anti-L3T4 plus complement (■-■) anti-Lyt2 plus complement (◇-◇) or with complement only (◆-◆). (□-□) shows no treatment control group.

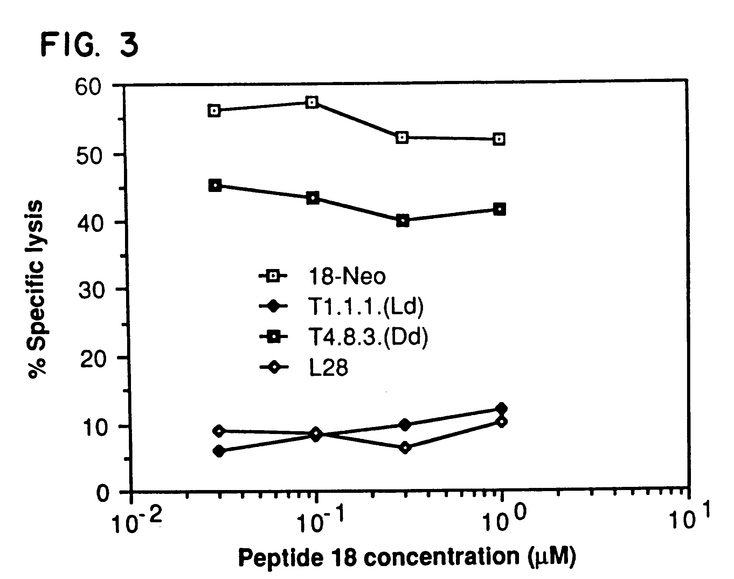

Fig. 3 shows the CTL specific for peptide Env-K₁ are restricted by the class I molecule D^{d} 5 x 10⁴ effector cells from the long-term line were cultured with 5 x 10³ ⁵¹Cr-labelled targets in the presence of various concentrations of Env-K₁. T1.1.1. (◆-◆) and T4.8.3. (■-■) indicate L-cell (H-2^{k} transfectants expressing L^{d} and D^{d}, respectively. Neo-gene transfected 3T3 fibroblasts, 18-Neo, (K^{d},L^{d},D^{d}) (□-□) were used as a positive control and neo-gene transfected L-cells, L28, (K^{k},D^{k}) (◇-◇) as a negative target control. Specific lysis in the absence of peptide was -0.2, 0.3, 1.5, and 0.02% for the 4 targets, respectively. Spontaneous release ranged from 14.6 to 16.6%.

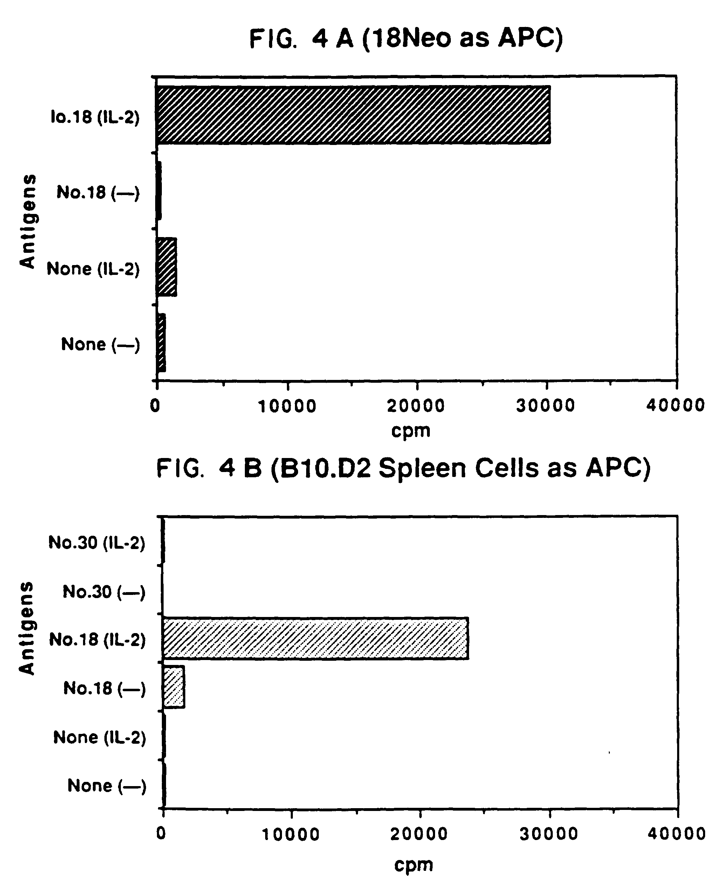

Fig. 4 shows the stimulation of proliferation of the CTL LINE by Env-K₁ in the presence of IL-2. Panel 4A shows 1 x 10⁴ LINE cells were stimulated with 0.1 µM Env-K₁ (Peptide No. 18) or no peptide with or without 10% IL-2 (the same source as for making and maintaining the LINE) in the presence of 2 x 10⁴ mitomycin-C treated (50 µg/ml, 37°C, 30 min) 18Neo transfectants (H-2^{d}) in 200 µl of medium in 96-well flat-bottom culture plates. After 3-days, wells were pulsed with 1µ Ci of ³H-thymidine (6.7 Ci/mmole) and an additional 16 hr later, ³H activity was measured by β-scintillation counting. Panel 4B shows 1 x 10⁴ LINE cells were stimulated with 0.1 M Env-K₁ (Feptide No. 18) or peptide No.30 or no peptide with or without 10% IL-2 in the presence of 4 x 10⁵ irradiated (3300 rad) B10.D2 spleen cells (SC) (H-2^{d} in 200 µl of medium in 96-well flat-bottom culture plates. Thymidine incorporation was measured as in panel A.

### DETAILED DESCRIPTION OF INVENTION

The above and various other objects and advantages of the present invention are achieved by an isolated, synthetic oligopeptide, Env-K₁, having about 15 amino acid residues in the following sequence: Arg - Ils - Gin - A rg - Gly - Pro - Gly - A rg - Ala - Phe - Val - Thr - Ile - Gly - Lys , or a functionally equivalent variant thereof.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned hereunder are incorporated herein by reference. Unless mentioned otherwise, standard procedures or methodology, well known to one of ordinary skill in the are, are followed.

The term "variant" or "functionally equivalent variant" as used herein means that the oligopeptide Env-K₁ of the present invention can be modified by deletion, addition, substitution or derivatization of the amino acid residues described herein, in any suitable manner so long as the resulting peptide acts in a functional manner similar to that of the Env-K₁ for any HIV isolates. All such variants or derivatives of the Env-K₁ peptide of the present invention are included herein as being functional equivalents thereof, it being understood that with the modern techniques of chemical synthesis and manipulations several variations of the amino acid sequence of Env-K₁, while maintaining the essential biological properties thereof, are within the skill of an ordinary artisan in the field to which this invention belongs.

Given the amino acid sequence of Env-K₁, the peptide is easily and conveniently synthesized employing the commercially available peptide synthesizers (such as Applied Biosystems Model 430A) well known to one of ordinary skill in the art.

The details of the materials and the methods are now described.

Mice: BALB/c (H-2^{d}) and C3H/HeN (H-2^{k} mice were obtained from the Jackson Laboratories (Bar Harbor, ME). BALB.K (H-2^{k} and BALB/c mice used in certain experiments were bred in the colony maintained at NIH. Mice were used at 6-12 weeks of age.

Recombinant vaccinia viruses: vSC-8 (recombinant vaccinia vector containing the bacterial lacZ gene), and vSC-25 (recombinant vaccina vector expressing the HIV env glycoprotein gp 160 of the HTLV III_{B} isolate without other HIV structural or regulatory proteins) were the same as described by Chakrabarti, et al., Nature, 320: 535-537 (1986).

Transfectants: An EcoRV fragment containing the region of HIV clone BH.8 encoding gp 160 used to construct vSC-25 was cloned into the SV40-drived expression vector pcEXV-3. This DNA was co-transfected along with pSV2neo into either BALB/c-3T3 (H-2^{d} or DAP.3 L cell (H-2^{k}) fibroblasts using a standard calcium phosphate precipitation method. Transfectants were also prepared with PSV2neo alone. Individual clones were picked after selection G418 (Geneticin, GIBCO), expanded, and tested for expression of the gp 160 gene by RNA hybridization using the EcoRB fragment labelled by random hexamer priming as probe. T1.1.1 and T4.8.3 are L cell transfectants expressing L^{d} and D^{d} class I MHC molecules, respectively.

Monoclonal antibodies (mAb): The following mAb were used: Anti-Lyt2 (3.155; rate IgM) and anti-L3T4 (RL172.4; rat IgM)

CTL generation: Mice were immunized i.v. with 10%⁷PFU of recombinant vaccinia viruses. Three to 14 weeks later, immune spleen cells (5x10⁶/ml in 24-well culture plates in complete T-cell medium) were restimulated for 6 days in vitro with either 2.5 x 10⁶/ml of recombinant vaccinia virus-infected syngeneic spleen cells (1 hr, 37°, MOI of 10:1) or 2 x 10⁵/ml of gp 160-gene transfected, MHC-identical fibroblasts. A long term CTL line was also generated by repetitive stimulation of immune cells with mitomycin- treated, gp 160 gene-transfected fibroblasts and 10% Con-A supernatant-containing medium.

CTL assay: After culture for 6 days, cytolytic activity of the restimulated cells was measured by the method of Bennink, et al., Nature, 311:578-579 1984) using a 6 hr assay with various ⁵¹Cr-labelled targets were mixed with various concentrations of peptide at the beginning of the assay. The percent specific ⁵¹Cr release was calculated as 100 (experimental release - spontaneous release)/(maximum release - spontaneous release). Maximum release was determined from supernatants of cells that were lysed by addition of 5% Triton-X 100. Spontaneous release was determined from target cells incubated without added effector cells.

Peptide synthesis: Synthetic peptides 13 to 23 residues long were prepared using the multiple simultaneous peptide method of solid-phase peptide synthesis, in polyproylene mesh "tea-bags" as described by Houghten, Proc. Natl. Acad. Sci., U.S.A., 82:5131-35 (1985). Conventional blocked BOC amino-acids and HF cleavage were used. Peptides were desalted by reverse phase chromatography on C-18 "sep-Pak" columns (Waters) and analyzed by HPLC. They were prescreened for lack of nonspecific toxicity or mitogenicity in a lymph node T cell proliferation assay.

Alternatively, Env-K₁ is also synthesized by commercially available peptide synthesizers, such as Applied Biosystems Model 430A.

### Demonstration that peptide Env-K1 is an immunodominant epitope for HIV envelope-specific CTL, stimulates proliferation of HIV-specific CTL and sensitizes targets to be killed by these CTL.

Conditions for eliciting murine CTL specific for HIV gp 160. The ability of recombinant vacinia viruses to prime and stimulate CTL specific for the products of inserted viral genes was used to generate CTL specific for HIV gp 160 using BALB/c (H-2^{d})mice. Specificity for gp 160 was found at three levels--lymphocyte priming, re-stimulation, and effector function (Table 1). Thus, only the recombinant vaccinia virus expressing gp 160 (vSC-25), no the recombinant vaccina virus containing the bacterial lacZ gene (vSC-8), could prime mice for development of CTL able to kill the gp 160-expressing fibroblast, 15-12 (compare groups 2 and 4, and groups 5 and 6). Likewise, only syngeneic vSC-25 infected cells, or the gp 160-expressing transfectant 15-12, but not cells infected with control vaccinia virus vSC-8, could restimulate immune T cells in vitro to kill the specific target (groups 4 and 6 vs group 3). CTL from gp 160-primed and restimulated spleen cells preferentially killed gp-160 expressing targets vs control H-2 matched targets (groups 4 and 6). The modest but significant nonspecific killing of control targets (18 neo) by vSC-25 restimulated spleen cells was absent when restimulation was done with the transfectant rather than vaccinia-infected cells. Vaccinia (vSC-25)-infected targets served as a positive control. Finally, a long-term line of CTL effectors specific for gp-160 expressing targets was established by repetitive stimulation of spleen cells from vSC-25-immunized mice with 15-12 transfectants and Con A supernatant (group 7).

Identification of an immunodominant CTL epitope on gp 160. A series of 41 overlapping peptides in 17 clusters covering 46% of the HIV gp 160 env protein sequence, selected by use of the amphipathicity algorithm for searching for immunodominant helper T-cell epitopes but including some additional regions of the sequence as controls, was synthesized to study helper epitopes and was used to search for CTL epitopes as well. To identify the T-cell epitopes recognized by gp 160-specific BALB/c CTL, various concentrations of peptide were added to the effectors and ⁵¹Cr-labelled fibroblast tumor targets at the start of the assay culture. The cytotoxic activity of three types of effector cells was measured: vSC-25-immune spleen cells stimulated once in vitro either with the 15-12 transfectant or with vSC-25 infected-autologous cells (presumably polyclonal effector populations), and the long-term CTL line (possibly an oligoclonal population). The results show that among the various peptides tested, only one (No. 18, henceforth called Env-K₁) could sensitize H-2^{d} target cells for high levels of specific killing by all three types of effectors (Table 2). Therefore, it is concluded that the sequence contained in peptide Env-K₁ contains an immunodominant epitope of gp 160 for cytotoxic cells of the H-2^{d} haplotype. Some other peptides (No. 21, No. 41, No. 42, No. 47) appeared to marginally sensitize target cells, but these effects were insignificant compared to the activity of Env-K₁. Env-K₁ was found to be surprisingly potent in sensitizing targets for being killed by HIV-specific CTL at very low concentrations of the peptide. For instance, at 0.1 µM peptide, the killing was still on a plateau, and only at 0.01 µM peptide did the killing fall to half-maximal (Fig. 1). All of the active peptides were identified as capable of forming an amphipathic helix.

Surface phenotype and Class I MHC restriction of the anti-gp 160 CTL. Treatment of the CTL effector cells with anti-Lyt 2 monoclonal antibody plus rabbit complement, but not anti-L3T4 antibody plus complement or complement alone led to loss of killing activity on either the 15-12 fibroblasts transfected with the gp 160 gene or the control fibroblasts incubated in the presence of Env-K₁ (Fig. 2). These data show that the effector cells which recognize and kill HIV envelope gp 160 protein-expressing cells in our system, including those specific for Env-K₁, are conventional Lyt2+L3T4-(CD8+CD4-) CTL.

The fibroblasts used as targets in these experiments express class I but not class II MHC gene products. Therefore, the gp 160-specific CTL capable of lysing the 15-12 transfectants or the peptide-bearing fibroblasts were likely to be class I MHC molecule-restricted, as is usual for Lyt2+ effected T cells. In the H-2^{d} haplotype there are three major class I molecules known to be used as restriction elements, K^{d},D^{d}, and L^{d}. To determine which of these was involved in recognition of Env-K₁, we used two L-cell (H-2^{k} transfectants, T1.1.1 and T4.8.3, expressing L^{d} or D^{d}, respectively Figure 3 demonstrates that recognition of Env-K₁ is restricted by the class I D^{d} molecule, not the L^{d} molecule.

H-2k mice appear to be low responders for cytotoxic T cells to gp 160. We investigated a second haplotype, H-2^{k}, using the same protocol as in the case of H-2^{d}. L cells (H-2^{k}) were similarly transfected with and shown to express the gp 160 gene, but no cytotoxic activity could be detected against these targets using spleen cells from vSC-25-immune BALB.K (H-2^{k}) mice stimulated in vitro with syngeneic vSC-25-infected spleen cells or with the L-cell transfectant itself (Table 3). Similarly, none of the peptides tested produced targets that could be killed by such effector cells (Table 2). Despite the lack of gp 160-specific CTL activity, the BALB.K mice were effectively primed and restimulated as they produced potent vaccinia-specific CTL (Table 3, from the same experiment as Table 2). Similar results were obtained with C3H (H-2^{k}) mice (Table 3 and data not shown). Because BALB.K mice are congenic to BALB/c responder mice but bear the H-2^{k} haplotype, the difference in responsiveness is MHC-linked. These results suggest that H-2^{k} is a CTL low responder to gp 160 as assayed under the present conditions, in contrast to H-2^{d}, which is a high responder haplotype.

Demonstration that Env-K1 stimulates proliferation of CTL specific for the envelope of HIV. The peptide Env-K₁ was tested for its ability to stimulate proliferation in vitro of the CTL line demonstrated above to be specific for the envelope of HIV. The data in Figure 4 demonstrate that Env-K₁ stimulates proliferation of these HIV-specific CTL cells, as measured by incorporation of tritiated thymidine into DNA, and that this stimulation is enhanced in the presence of the lymphokine interleukin-2(IL-2).

Env-K1 as a diagnostic reagent. If a person suspected of being exposed to AIDS is tested for antibodies to HIV and is determined to be seronegative, it is still possible that that person is carrying the virus but has not made antibodies, because a certain percentage of exposed individuals do not develop antibodies for a significant period of time after exposure, and some may never develop antibodies. Nevertheless, that person may have developed a cell-mediated immune response in the form of CTL specific for the envelope of the virus. Because that person's CTL are specific not only for the virus but also for that individual's major histocompatibility complex (MHC) antigens, it is not possible to test for these on transfected tumor targets as was done in the case of the mice, unless one is lucky enough to have transfected tumor that shares MHC (HLA) molecules with the individual to be tested. Because there are so many human HLA types, iot is not feasible to have transfected cells of every type available. Although it is possible to produce a transformed tumor line from the individual and transfect it with HIV genes, this is a very difficult, time-consuming, laborious process and would not be feasible to do for large numbers of people. Infecting the individual's cells with HIV requires the appropriate cell type that can be infected, and would be hazardous for laboratory workers to handle the concentrated virus. Use of the vaccinia recombinant described above may give many false positive results since most individuals in the U.S. born before 1972 were immunized with vaccinia as a smallpox vaccination, and so would have vaccinia-specific CTL. Purified proteins are generally not taken up by cells in such a way as to make them targets for CTL. However, small peptides such as Env-K₁ are capable of sensitizing targets for CTL, as we have shown herein above. Therefore, it would be relatively simple to use Env-K₁ as a diagnostic reagent to test for the presence of HIV-specific CTL in the peripheral blood of an individual. This can be achieved by standard procedures; first, produce PHA or ConA blasts of the peripheral blood lymphocytes of the individual to be tested, label these with ⁵¹Cr as indicated herein above, incubate these with the peptide Env-K₁ under standard culture conditions similar to those given herein above, but modified for human CTL assays, and add fresh peripheral blood lymphocytes from the same individual. After about 6 hours, one measures the amount of ⁵¹Cr released into the culture medium, and compares this with controls treated identically but without any peptide, or with a control peptide, without any fresh lymphocytes, and with the maximum ⁵¹Cr release produced by detergent lysis of the target cells. If there is specific release, the individual can be judged to be carrying the HIV virus, even though no antibodies could be detected.

Env-K1 as a prognostic reagent. Although it is not yet known which arms of the immune system, if any, are protective against an HIV infection, CTL is likely to play a significant role for the reasons outlined herein above. Therefore, it is possible that seropositive but still clinically healthy individuals, who have CTL able to kill virus infected-cells, would be less likely to develop clinical AIDS than those without such CTL. Therefore, Env-K₁ could be used to test seropositive individuals at various stages of disease by the methods indicated in the preceding section on diagnostic use, and if there exists a significant correlation between healthy clinical status and presence of CTL specific fcr Env-K₁ this will serve as a prognostic test to predict which seropositive individuals are at greater or lesser risk for developing clinical immunodeficiency or full-blown AIDS.

Env-K1 as a therapeutic agent: Because the peptide Env-K₁, can stimulate the proliferation and expansion of a population of HIV-specific CTL, Env-K₁ can be used as a therapeutic agent to enhance the immune response of a patient already infected with the virus. Such therapeutic modality could be better employed in conjunction with other therapeutic or preventive strategies for the control of the disease.

An advantage of the present invention is that, for the first time, a pure CTL-stimulatory antigenic product has been identified and synthesized in vitro without any contact with dangerously live infectious virus or product derived from such virus, that it has none of the risks involved in the handling of or exposure to HIV or HIV-derived products.

Another significant aspect of the present invention is that the peptide of the present invention makes it possible to detect and prevent cell-mediated HIV infection which may not produce any antibodies at all. Hence, those tests _which depend on the detection of anti-HIV antibodies in a body sample, would be of no use in those cases of HIV infection where antibodies are not produced. The antigen of the present invention now provides means of detecting cell-transmitted HIV infection in the absence of antibodies, which was not heretofore possible by the currently employed antibody detecting tests. Likewise, when the mode of infection is cell-transmission as described herein above, neutralizing antibodies would not be protective. Hence, a vaccine using the Env-K₁ as an antigen, for the first time, provides a method of preventing HIV infection, not through antibody-neutralization mechanism, but by killing HIV-infected cells by proliferating CTL's produced through antigenic stimulantion of T cells by Env-K₁.

A pharmaceutical composition including a vaccine in accordance with the present invention comprises an effective antigenic or therapeutic amount of Env-K₁ to produca CTL's and a pharmaceutically acceptable carrier such as physiological saline, non-toxic, sterile buffer and the like. Of course, additives such as preservatives, sterilants, adjuvants and the like, well known to one of ordinary skill in the art, could also be included in the pharmaceutical composition to maintain or increase the efficacy of the composition.

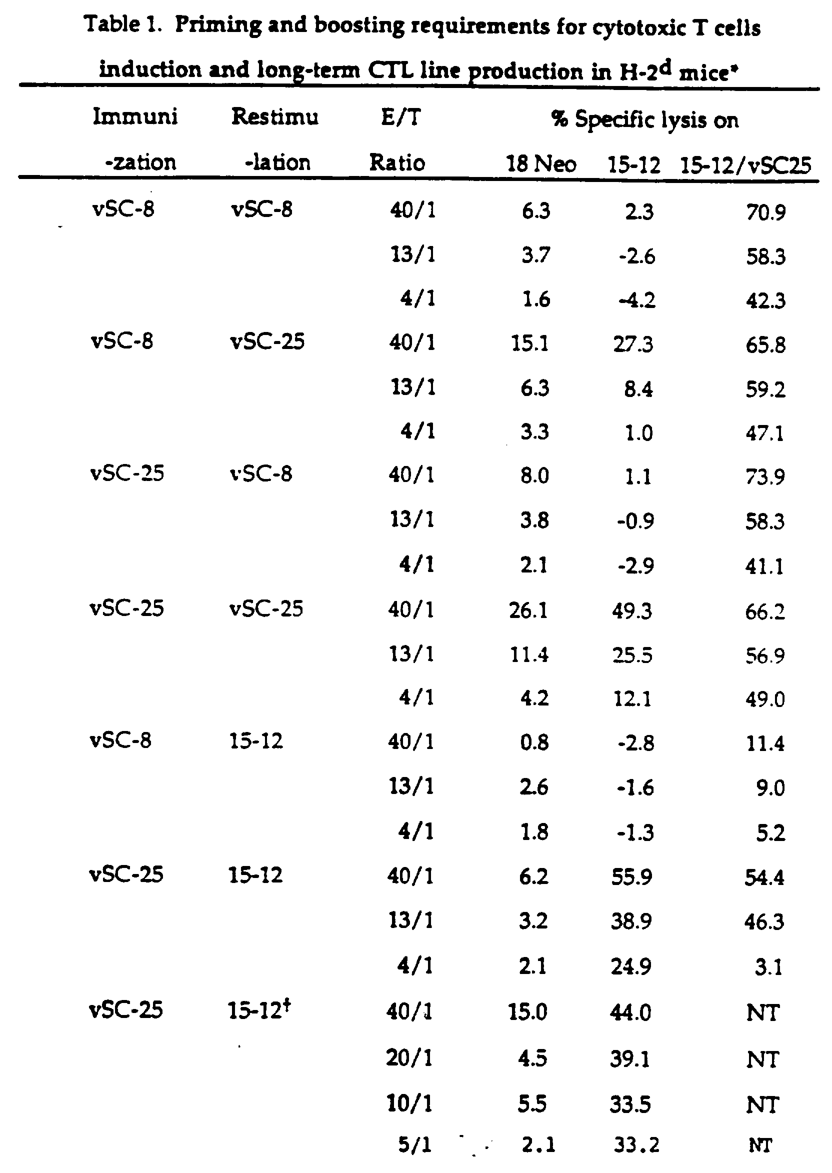

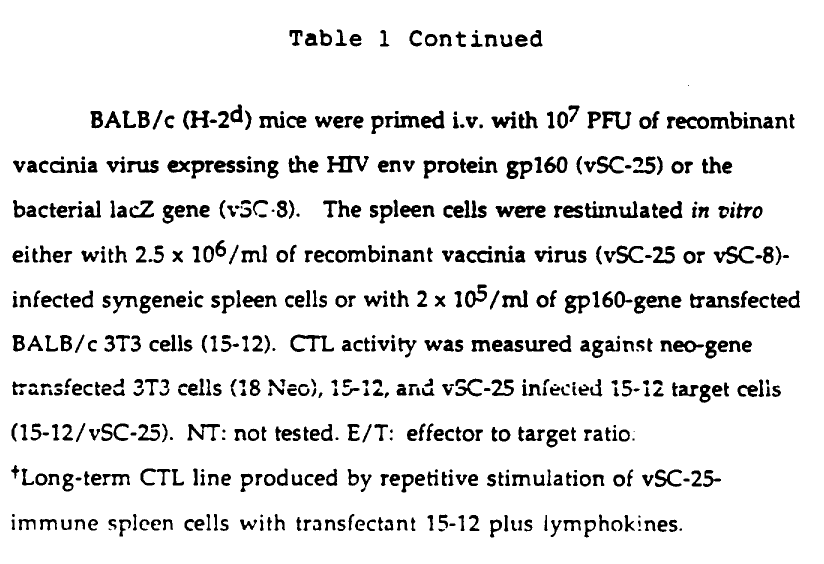

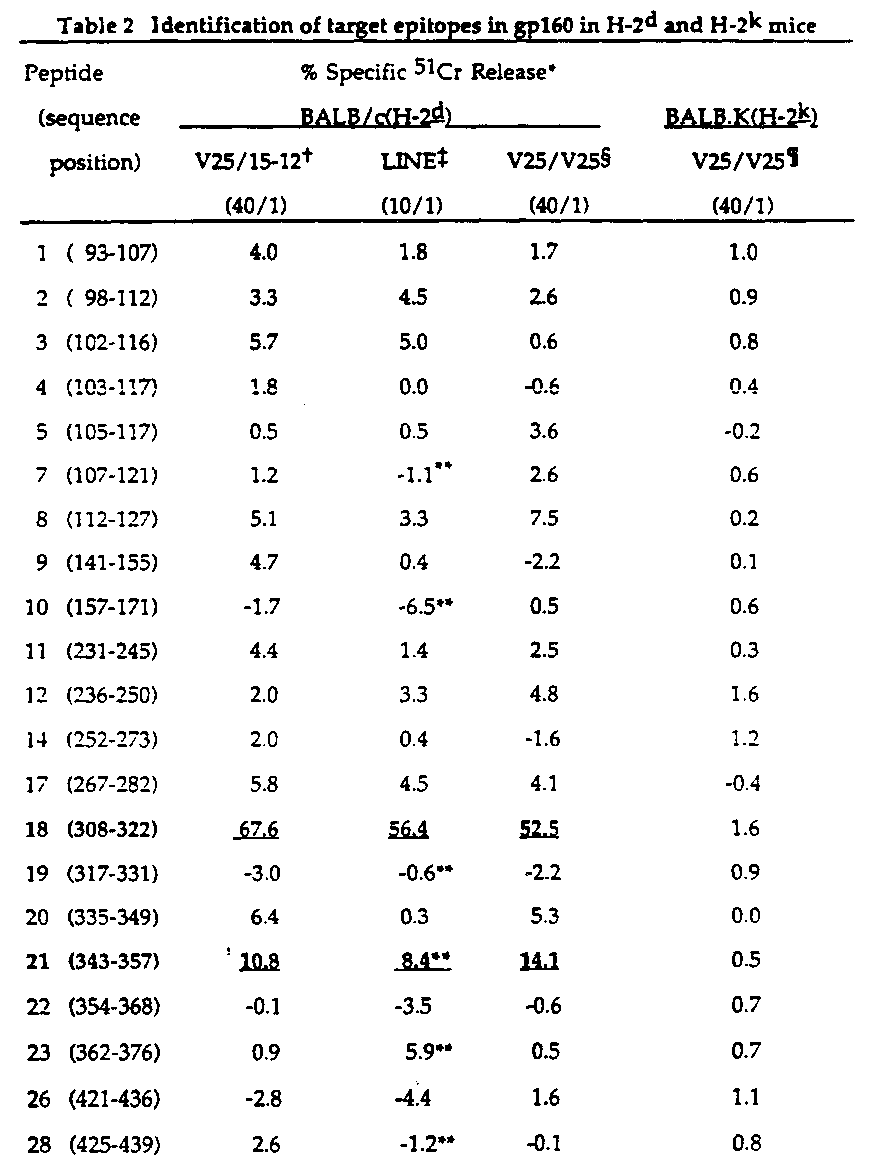

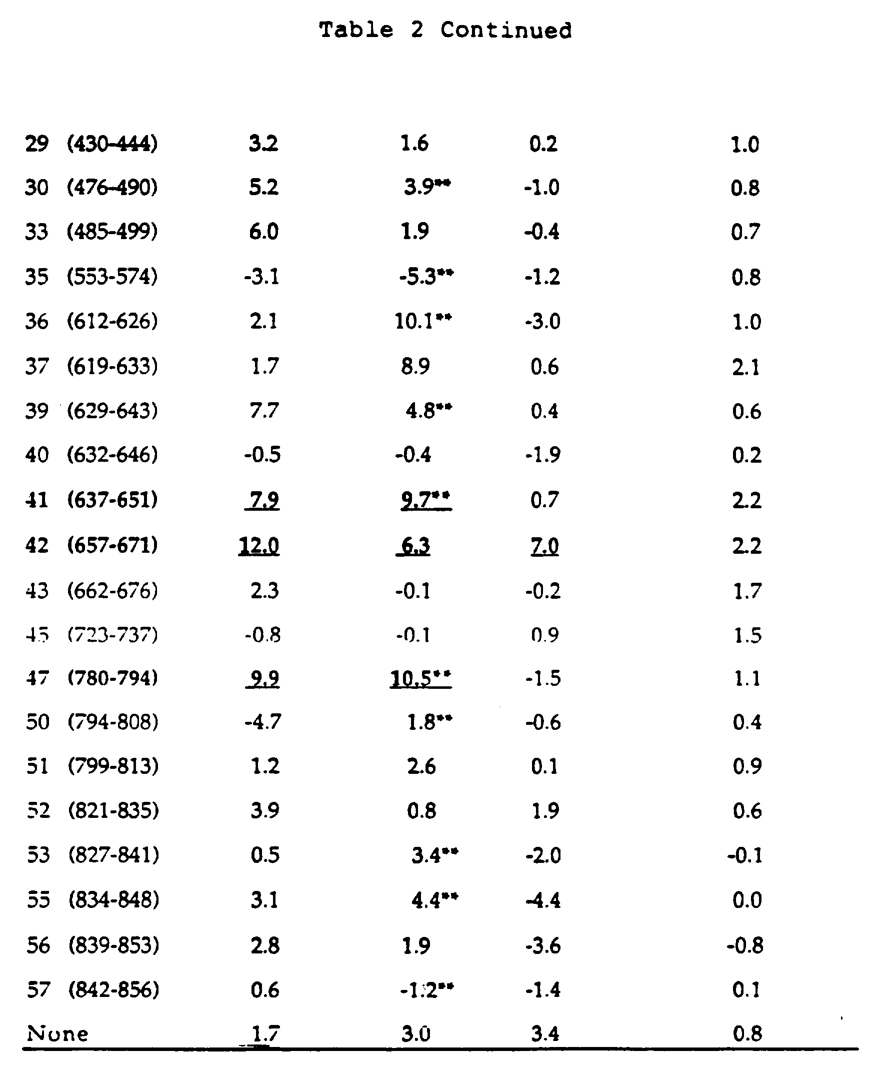

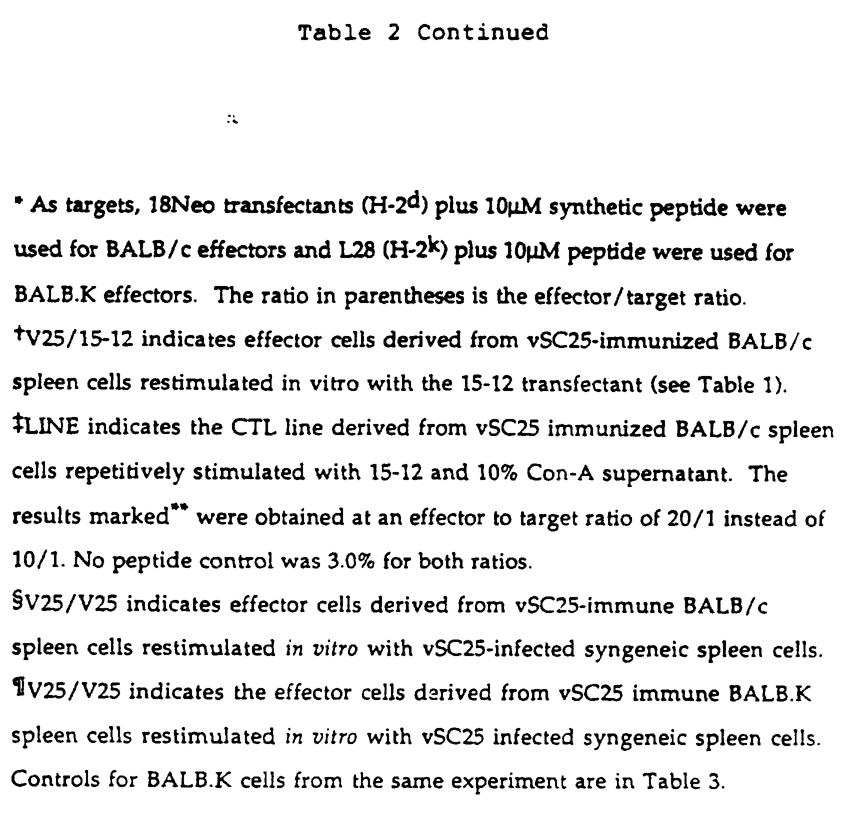

**Table 3.**

| H-2^{k} (BAL.BK and C3H) mice fail to respond with CTL specific for gp160 HIV *env* transfectants expressing the HIV envelope protein.* | | | | | | |
|---|---|---|---|---|---|---|
| Immunization | Restimulation | E/T | | ⁵¹Cr release (%) | | |
| | | Ratio | 25-20 | L28 | 25-20/V25 | L28/V25 |
| vSC-25 | vSC-25 inf. | 80/1 | 1.2 | 0.5 | 56.2 | 57.7 |
| (BALB.K) | | 40/1 | 1.3 | 0.8 | 47.3 | 44.4 |
| | | 20/1 | 0.7 | 0.6 | 39.4 | 39.0 |
| | | | | | | |
| vSC-25 | vSC-25 inf. | 80/1 | 3.7 | 1.7 | N.T. | 60.1 |
| (C3H) | | 40/1 | 1.9 | 0.4 | N.T. | 51.9 |
| | | 20/1 | -0.1 | 0.1 | N.T. | 41.5 |
| | | | | | | |
| vSC-25 | 25-20 | 60/1 | 11.8 | 15.7 | N.T. | 35.7 |
| (C3H) | | 20/1 | 6.8 | 8.5 | N.T. | 16.7 |
| | | 7/1 | 3.9 | 2.3 | N.T. | 5.6 |

BALB.K (H-2^{k}) or C3H (H-2^{k}) spleen cells immunized with vSC-25 were boosted in vitro with either the gp160-expressing L-cell transfectant, 25-20, or vSC-25-infected syngeneic spleen cells. After culture for 6 days, cytolytic activity of the restimulated cells was measured against 25-20, or neo-gene transfected L-cells (H-2^{k}), L28, as a control target or vSC-25-infected 25-20 (25-20/V25) and L28 (L28/V25), as positive control targets.

## Claims

1. A peptide selected from the group of peptides consisting of the Env-K1 peptide and peptides that are functionally equivalent variants of the Env-K1 peptide, wherein said variants can be derived from the Env-K1 peptide by addition, deletion, substitution, or derivatization of at least one amino acid thereof and wherein the Env-K1 peptide has the following amino acid sequence: Arg-Ile-GIn-Arg-Gly-Pro-Gly-Arg-Ala-Phe-Val-Thr-Ile-Gly-Lys; provided that the following peptides having the sequence: Ile-Arg-Ile-Gln-Arg-Gly-Pro-Gly-Arg-Ala-Phe-Val-Thr-IIe-GIy-Lys-Ile-Gly-Asn-Met-Arg-Gln-Ala-His-Cys; Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg; and Thr-Arg-Pro-Asn-Asn-Asn-Thr-Arg-Lys-Ser-Ile-e-Ile-f-f-Gly-Pro-Gly in which e is arg or tyr and f is gln, arg or omitted, are excluded.

2. A pharmaceutical composition for inducing cytotoxic lymphocytes that specifically recognize cells that express human immunodeficiency virus antigens, comprising a pharmaceutically acceptable carrier and a peptide of Claim 1.

3. A diagnostic or prognostic kit for performing an assay for detecting cell-mediated human immunodeficiency virus infection, comprising:
(a) a peptide of claim 1;
(b) a mitogen that is effective for stimulating peripheral blood lymphocytes;
(c) a label for detecting the lysis of peripheral blood lymphocytes; and
(d) instruction material for performing said prognostic or diagnostic assay.

4. Use of the peptide of claim 1 for preparing a pharmaceutical composition for preventing or treating human immunodeficiency virus infection in a susceptible host, wherein said composition contains a sufficient amount of said peptide to induce in said host cytotoxic lymphocytes that selectively kill only cells that are infected with said human immunodeficiency virus.

5. A method for diagnosing infection of a susceptible host with a human immunodeficiency virus (HIV), comprising the steps of:
(a) incubating autologous labelled peripheral blood lymphocytes (PBL) from said host with a peptide of claim 1 in order to activate T cells in said PBL that are infected with said virus, wherein said lymphocytes have been stimulated with a mitogen prior to incubation and said label permits detection of the percentage of said PBL that lyse;
(b) adding fresh PBL from said host and continuing incubation for a time sufficient for any cytotoxic lymphocytes to lyse said activated T cells in said labelled PBL;
(c) detecting the presence of (HIV) infected cells by detecting lysed infected T-cells by measuring the number of labelled PBL that lyse in step (b) minus the number of stimulated PBL that lyse after incubation in the absence of said peptide and minus the number of labelled PBL that lyse after incubation in the presence of said peptide but in the absence of fresh PBL.

6. The method of claim 5, wherein said PBL are labelled with ⁵¹Cr and lysis is detected by release of said ⁵¹Cr.

7. The method of claim 6, wherein said mitogen is selected from the group consisting of phytohemagglutinin and concanavalin A.

## Patentansprüche

1. Peptid, ausgewählt aus der Gruppe von Peptiden bestehend aus dem Env-K1-Peptid und Peptiden, die funktionell äquivalente Varianten des Env-K1-Peptids sind, wobei die Varianten von dem Env-K1-Peptid durch Addition, Deletion, Substitution oder Derivatisierung wenigstens einer Aminosäure desselben hergeleitet sein können und wobei das Env-K1 Peptid die folgende Aminosäuresequenz hat: Arg-Ile-Gln-Arg-Gly-Pro-Gly-Arg-Ala-Phe-Val-Thr-Ile-Gly-Lys; vorausgesetzt, daß die folgenden Peptide, die die Sequenz: Ile-Arg-Ile-Gln-Arg-Gly-Pro-Gly-Arg-Ala-Phe-Val-Thr-Ile-Gly-Lys-Ile-Gly-Asn-Met-Arg-Gln-Ala-His-Cys; Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg; und Thr-Arg-Pro-Asn-Asn-Asn-Thr-Arg-Lys-Ser-Ile-e-Ile-f-f-Gly-Pro-Gly, in welcher e Arg oder Tyr ist und f Gln, Arg oder weggelassen ist, haben, ausgeschlossen sind.

2. Pharmazeutische Zusammensetzung zur Induzierung zytotoxischer Lymphozyten, die spezifisch Zellen erkennen, die Human-Immunschwächevirus-Antigene exprimieren, umfassend einen pharmazeutisch akzeptablen Träger und ein Peptid nach Anspruch 1.

3. Diagnostisches oder prognostisches Kit zur Durchführung einer Untersuchung zur Entdeckung von zellvermittelter Human-Immunschwächevirusinfektion, umfassend:
(a) ein Peptid nach Anspruch 1;
(b) ein Mitogen, das zur Stimulierung von Peripherieblut-Lymphozyten wirksam ist;
(c) eine Markierung zur Entdeckung der Lyse von Peripherieblut-Lymphozyten; und
(d) Instruktionsmaterial zur Durchführung der prognostischen oder diagnostischen Untersuchung.

4. Verwendung des Peptids nach Anspruch 1 zur Herstellung einer pharmazeutischen Zusammensetzung zum Verhindern oder Behandeln einer Human-Immunschwächevirusinfektion in einem dafür empfänglichen Wirt, wobei die Zusammensetzung eine ausreichende Menge des Peptids enthält, um in dem Wirt zytotoxische Lymphozyten zu induzieren, die selektiv nur Zellen töten, die mit dem Human-Immunschwächevirus infiziert sind.

5. Verfahren zur Diagnose der Infektion eines dafür empfänglichen Wirtes mit einem Human-Immunschwächevirus (HIV), umfassend die Schritte:
(a) Inkubieren von autolog markierten Peripherieblut-Lymphozyten (PBL) von dem Wirt mit einem Peptid nach Anspruch 1, um T-Zellen in den PBL zu aktivieren, die mit dem Virus infiziert sind, wobei die Lymphozyten vor der Inkubation mit einem Mitogen stimuliert wurden und die Markierung die Erfassung des Prozentsatzes derjenigen PBL, die lysieren, erlaubt;
(b) Hinzugeben von frischen PBL von dem Wirt und Fortführen der Inkubation für eine Zeitdauer, die ausreicht, daß zytotoxische Lymphozyten die aktivierten T-Zellen in den markierten PBL lysieren;
(c) Erfassung der Anwesenheit von (HIV)-infizierten Zellen durch Erfassung von lysierten infizierten T-Zellen durch Messung der Anzahl von markierten PBL, die in Schritt (b) lysieren, minus der Anzahl der stimulierten PBL, die nach der Inkubation in Abwesenheit von dem Peptid lysieren, und minus der Anzahl von markierten PBL, die nach der Inkubation in der Anwesenheit des Peptids, aber in der Abwesenheit von frischen PBL lysieren.

6. Verfahren nach Anspruch 5, wobei die PBL mit ⁵¹CR markiert werden und die Lyse durch die Freisetzung von ⁵¹Cr erfaßt wird.

7. Verfahren nach Anspruch 6, wobei das Mitogen ausgewählt ist aus der Gruppe bestehend aus Phythämagglutinin und Concanavalin A.

## Revendications

1. Peptide choisi dans le groupe de peptides formé par le peptide Env-K₁ et les peptides qui sont des variantes fonctionnellement équivalentes du peptide Env-K₁, dans lequel lesdites variantes peuvent être dérivées du peptide Env-K₁ par addition, délétion, substitution, ou dérivation d'au moins un acide aminé de celui-ci et dans lequel le peptide Env-K₁ a la séquence d'acides aminés suivante : Arg-Ile-Gln-Arg-Gly-Pro-Gly-Arg-Ala-Phe-Val-Thr-Ile-Gly-Lys; à condition que les peptides suivants qui ont la séquence : Ile-Arg-Ile-Gln-Arg-Gly-Pro-Gly-Arg-Ala-Phe-Val-Thr-Ile-Gly-Lys-Ile-Gly-Asn-Met-Arg-Gln-Ala-His-Cys; Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg; et Thr-Arg-Pro-Asn-Asn-Asn-Thr-Arg-Lys-Ser-Ile-e-Ile-f-f-Gly-Pro-Gly dans laquelle e est arg ou tyr et f est gln, arg, ou omis, soient exclus.

2. Composition pharmaceutique destinée à induire des lymphocytes cytotoxiques qui reconnaissent spécifiquement les cellules qui expriment les antigènes du virus d'immunodéficience humaine, comprenant un véhicule pharmaceutiquement acceptable et un peptide selon la revendication 1.

3. Trousse de diagnostic ou de pronostic destinée à réaliser un test de dépistage d'une infection par le virus d'immunodéficience humaine à médiation cellulaire, comprenant :
(a) un peptide selon la revendication 1 ;
(b) un mitogène qui est efficace pour stimuler les lymphocytes du sang périphérique ;
(c) un marqueur destiné à détecter la lyse des lymphocytes du sang périphérique ; et
(d) des instructions pour réaliser ledit test de pronostic ou de diagnostic.

4. Utilisation du peptide selon la revendication 1 pour préparer une composition pharmaceutique destinée à prévenir ou à traiter une infection par le virus de l'immunodéficience humaine chez un hôte prédisposé, dans laquelle ladite composition contient une quantité suffisante dudit peptide pour induire chez ledit hôte des lymphocytes cytotoxiques qui tuent sélectivement uniquement les cellules qui sont infectées avec ledit virus d'immunodéficience humaine.

5. Procédé destiné à diagnostiquer chez un hôte prédisposé une infection par le virus de l'immunodéficience humaine (VIH) comprenant les étapes suivantes :
(a) incubation de lymphocytes du sang périphérique (PBL) autologues, marqués, prélevés chez ledit hôte avec un peptide selon la revendication 1 dans le but d'activer les lymphocytes T dans lesdits PBL qui sont infectés avec ledit virus, dans lequel lesdits lymphocytes ont été stimulés avec un mitogène avant incubation et ledit marqueur permet de détecter le pourcentage desdits PBL qui se lysent ;
(b) addition de PBL frais provenant dudit hôte et poursuite de l'incubation pendant un temps suffisant pour que les lymphocytes cytotoxiques lysent lesdits lymphocytes T activés dans lesdits PBL marqués ;
(c) détection de la présence de cellules infectées (VIH) par détection des lymphocytes T infectés lysés en mesurant le nombre de PBL marqués qui se lysent dans l'étape (b) moins le nombre de PBL stimulés qui se lysent après incubation en l'absence dudit peptide et moins le nombre de PBL marqués qui se lysent après incubation en présence dudit peptide mais en l'absence de PBL frais.

6. Procédé selon la revendication 5, dans lequel les PBL sont marqués au radiochrome (⁵¹Cr) et la lyse est détectée par le relargage dudit ⁵¹Cr.

7. Procédé selon la revendication 6, dans lequel ledit mitogène est choisi dans le groupe formé par la phyto-hémagglutinine et la concanavaline A.
